# EUROPÄISCHE PATENTANMELDUNG

(11) **EP 3 075 449 A1**
(43) Veröffentlichungstag der Anmeldung: **05.10.2016**
(21) Anmeldenummer: 15162342.8
(22) Anmeldetag: 02.04.2015
(51) Int. Cl.: B01J 31/00, B01J 31/24, B01J 31/18, C07C 45/50, G01N 35/00, B01J 8/00

(54) **VERFAHREN ZUR UNTERSUCHUNG DER LANGZEITEIGENSCHAFTEN HOMOGENER KATALYSATORSYSTEME IM KONTINUIERLICHEN BETRIEB**

(71) Anmelder: Evonik Degussa GmbH, 45128 Essen (DE)
(72) Erfinder: Behr, Arno, 44137 Dortmund (DE); Gottschalk, Axel, 45527 Hattingen (DE); Kämper, Alexander, 44149 Dortmund (DE); Tlatlik, Stephen, 44309 Dortmund (DE); Franke, Robert, 45772 Marl (DE); Fridag, Dirk, 45721 Haltern am See (DE); Dyballa, Katrin Marie, 45657 Recklinghausen (DE); Geilen, Frank, 45721 Haltern am See (DE)

(57) **Zusammenfassung**

Die vorliegende Erfindung betrifft ein Verfahren zur Untersuchung der Langzeiteigenschaften homogener Katalysatorsysteme im kontinuierlichen Betrieb mit den Schritten: Einspeisen von Reaktanden in einen Reaktor; Zuführen einer definierten Ausgangsmenge Katalysator in den Reaktor; Erzeugen von mindestens zwei Phasen, umfassend eine katalysatorreiche Phase und eine produktreiche Phase; Abtrennen der produktreichen Phase von der katalysatorreichen Phase; Untersuchung des Katalysators, wobei die Untersuchung einen Vergleich zumindest einer Eigenschaft des Katalysators zum Zeitpunkt der erstmaligen Zuführung in den Reaktor und zu einem späteren Zeitpunkt beinhaltet.

## Beschreibung

Die vorliegende Erfindung betrifft ein Verfahren zur Untersuchung der Langzeiteigenschaften homogener Katalysatorsysteme im kontinuierlichen Betrieb sowie eine Anlage für die Durchführung eines solchen Verfahrens.

Eine Vielzahl industriell bedeutsamer Synthesen wird heutzutage homogen-katalytisch durchgeführt. Charakteristisch hierfür ist, dass das katalytische System homogen gelöst in dem Reaktionsgemisch vorliegt. Nach erfolgter Synthese werden entweder der Katalysator oder die Reaktionsprodukte aus dem Reaktionsgemisch abgetrennt, wobei der Katalysator zum Erreichen eines wirtschaftlichen und nachhaltigen Prozesses zur erneuten Nutzung in den Reaktor zurückgeführt wird. Dieser Rezirkulation kommt ökonomisch und ökologisch ein besonders hoher Stellenwert zu, wenn das verwendete Katalysatorsystem hochpreisige Metalle, insbesondere Seltenerd- oder Edelmetalle, enthält oder aufgrund seiner Eigenschaften eine Gefährdung für Mensch und/oder Umwelt darstellt.

Aufgrund der Bedeutung homogener Synthesen schreitet die Suche nach neuartigen und besseren Katalysatoren beständig fort. Im Mittelpunkt dieser Bemühungen stehen derzeit das Design neuer Katalysatoren und Liganden und die Untersuchung deren Eignung für die Katalyse gewünschter Reaktionen.

Für diese Zwecke beschreibt die WO 2012/041846 A1 einen SILP-Katalysator mit einer Zusammensetzung, die neben einem inerten porösen Trägermaterial und einer ionischen Flüssigkeit auch ein Metall der 9. Gruppe des Periodensystems der Elemente, einen phosphorhaltigen organischen Liganden und ein organisches Amin enthält.

Die WO 2014/056732 A1 beschreibt ein Verfahren zur Herstellung von Pentanal mittels Hydroformylierung unter Verwendung eines Katalysators mit Biphosphit-Liganden, bei dem der Betriebszeitraum mindestens 8000 Stunden andauert, wobei während des Betriebszeitraums auf eine Abscheidung fester Reaktionsrückstände aus dem Reaktor verzichtet wird.

Die EP 1 172 349 B1 beschreibt ein Verfahren zur Herstellung höherer Oxo-Aldehyde bzw. der entsprechenden Alkohole aus Olefinen, in dem der Katalysator aus dem flüssigen Reaktoraustrag entfernt und direkt oder nach einer Aufarbeitung in den Hydroformylierungsreaktor zurückgeführt wird.

Die Untersuchungen neuer Katalysatorsysteme sind derzeit auf kurzzeitige Versuche im nicht-industriellen Maßstab beschränkt, da bislang keine geeigneten Apparaturen und kein geeignetes Verfahren bekannt sind, mit denen langfristige und aussagekräftige Untersuchungen der verwendeten Katalysatorsysteme durchgeführt werden können. Die Langzeiteigenschaften entwickelter Katalysatorsysteme stellen sich daher oftmals erst im (quasi-)industriellen Betrieb heraus.

Es ist daher die Aufgabe der vorliegenden Erfindung, eine Vorrichtung und ein Verfahren bereitzustellen, das Untersuchungen der Langzeitleistungsfähigkeit homogen katalytischer Systeme in beliebigem Maßstab erlaubt und es ermöglicht, zutreffende Aussagen zu den Alterungs- und/oder Vergiftungsphänomenen der untersuchten Katalysatorsysteme im kontinuierlichen Prozess zu treffen.

Die Erfindung löst diese Aufgabe mit den Merkmalen der Ansprüche und insbesondere mit einem Verfahren zur Untersuchung der Langzeiteigenschaften homogener Katalysatorsysteme im kontinuierlichen Betrieb mit den Schritten
a) Einspeisen von Reaktanden in einen Reaktor,
b) Zuführen einer definierten Ausgangsmenge Katalysator in den Reaktor,
c) Erzeugen von mindestens zwei Phasen, umfassend eine katalysatorreiche Phase und eine produktreiche Phase,
d) Abtrennen der produktreichen Phase von der katalysatorreichen Phase,
e) Untersuchung des Katalysators, wobei die Untersuchung einen Vergleich zumindest einer Eigenschaft des Katalysators zum Zeitpunkt der Zuführung in den Reaktor unter

Schritt b) und zu einem späteren Zeitpunkt beinhaltet.

Bevorzugt wird im Laufe des kontinuierlichen Verfahrens der Katalysator nicht nachdosiert, d.h. dass die in dem Verfahren eingesetzte Gesamtmenge an Katalysator der in Schritt b) zugeführten Ausgangsmenge entspricht.

In dem erfindungsgemäßen Verfahren werden Reaktanden durch eine Einlassöffnung in den Reaktor eingespeist. Es können auch mehrere Einlassöffnungen vorgesehen sein, jeweils für ein Edukt oder mehrere Edukte. Durch dieselbe Einlassöffnung oder alternativ durch eine weitere Einlassöffnung wird der hinsichtlich seiner Langzeiteigenschaften zu untersuchende Katalysator dem Reaktor zugeführt, wobei der Katalysator bevorzugt homogen in einem Lösungsmittel gelöst in den Reaktor eingebracht wird.

Das erfindungsgemäße Verfahren weist weiterhin einen Schritt des Erzeugens von mindestens zwei Phasen auf, nämlich einer ersten katalysatorreichen Phase und einer zweiten produktreichen Phase. Dieser Schritt kann in dem Reaktor selber oder in einer optionalen dem Reaktor nachgeschalteten Trennstufe erfolgen. Für die Zwecke der Erfindung wird unter einer Trennstufe ein Behälter verstanden, in dem die katalysatorreiche Phase von der produktreichen Phase getrennt wird.

Der Schritt des Erzeugens von mindestens zwei Phasen kann einerseits automatisch, d.h. nur durch chemische Umsetzung der Reaktanden und ohne Hinzufügen eines Hilfsstoffes, stattfinden, nämlich dadurch, dass ein im Laufe der katalysierten Reaktion gebildetes Produkt Charakteristika aufweist, die von denen der Reaktanden so stark abweichen, dass sich eine neue Phase ausbildet, insbesondere durch die Ausbildung einer Mischungslücke oder im Falle flüssiger Reaktanden durch die Ausbildung einer Gasphase bzw. im Falle gasförmiger Reaktanden durch die Ausbildung einer Kondensatphase.

Alternativ kann das Erzeugen von mindestens zwei Phasen auch durch Änderung zumindest eines Betriebsparameters erfolgen, die sich unterschiedlich auf den Katalysator und das Produkt auswirkt, sodass sich beide in unterschiedlichen Phasen anreichern.

Weiter alternativ kann das Erzeugen von zumindest zwei Phasen auch durch die Zugabe eines ersten Hilfsstoffes zu dem Katalysator/Produkt-Gemisch, das optional noch Reaktanden enthält, erzwungen werden. Die Zugabe des ersten Hilfsstoffes zu dem Katalysator/Produkt-Gemisch führt zur Ausbildung einer neuen Phase, die bevorzugt Katalysator oder Produkt aufnimmt, sodass sich eine katalysatorreiche Phase und eine produktreiche Phase ausbilden, welche durch geeignete technische bzw. physikalische Maßnahmen voneinander getrennt werden können.

In einer Ausführungsform des erfindungsgemäßen Verfahrens dient der Reaktor, dem die Reaktanden sowie der zu untersuchende Katalysator zugeführt werden, sowohl als Reaktionsgefäß als auch als Trennstufe, d.h. die entsprechende Anlage für die Durchführung des Verfahrens weist keine dem Reaktor nachgeschaltete Trennstufe auf. In dieser Ausführungsform entstehen im Laufe der Reaktion zumindest eine produktreiche und eine katalysatorreiche Phase. Optional wird die Ausbildung dieser zumindest zwei Phasen durch Hinzufügen eines ersten Hilfsstoffes ausgelöst. Alternativ erfolgt die Ausbildung der zumindest zwei Phasen durch Änderung zumindest eines Betriebsparameters, beispielsweise Druck und/oder Temperatur. Bei thermomorphen Systemen kann dabei beispielsweise eine Mischungslücke entstehen. Bei anderen Systemen kann sich eine von der ersten Phase unterscheidbare Gasphase, eine Kondensatphase oder eine feste Phase ausbilden.

In jedem der vorgenannten Fälle, in denen der Reaktor sowohl als Reaktionsgefäß als auch als Trennstufe dient, wird die produktreiche Phase, die bevorzugt im Wesentlichen frei von Katalysator ist, anschließend durch eine Auslassöffnung aus dem Reaktor gelangt, während die den Katalysator enthaltende katalysatorreiche Phase im Reaktor verbleibt, sodass der Katalysator die Reaktion neu in den Reaktor eingespeister Reaktanden katalysieren kann.

In einer weiteren Ausführungsform des erfindungsgemäßen Verfahrens ist dem Reaktor zumindest eine Trennstufe nachgeschaltet, d.h. der Reaktor ist mit der zumindest einen Trennstufe über eine Leitung verbunden, durch die das im Laufe der Reaktion gebildete Gemisch aus bzw. mit Katalysator und Produkt aus dem Reaktor in die Trennstufe gelangt. Das Austreten des Gemisches aus dem Reaktor und dessen Eintreten in die Trennstufe erfolgt bevorzugt durch Pumpen oder durch Öffnen eines am Reaktorboden angeordneten Ventils, durch das das Gemisch in eine unterhalb des Reaktors angeordnete und mit dessen Auslassöffnung verbundene Trennstufe abgelassen wird, oder durch geeignete alternative Maßnahmen. In dieser Ausführungsform des Verfahrens erfolgt das Erzeugen der zumindest zwei Phasen insbesondere in der dem Reaktor nachgeschalteten Trennstufe, wobei bei mehreren dem Reaktor nachgeschalteten Trennstufen optional in jeder der Trennstufen eine Erzeugung von zumindest zwei Phasen erfolgen kann. Wie auch bei der Ausführungsform ohne dem Reaktor nachgeschaltete Trennstufen kann die Ausbildung bzw. Erzeugung der zumindest zwei Phasen durch Änderung eines Betriebsparameters, insbesondere durch eine Druckänderung und/oder durch eine Temperaturänderung ausgelöst werden. Alternativ kann dem in der Trennstufe befindlichen Gemisch, das den Katalysator und Produkt enthält, ein erster Hilfsstoff zugeführt werden, welcher eine neue Phase ausbildet, in der sich entweder der Katalysator oder das Produkt bevorzugt lösen. In jedem Fall entstehen in der Trennstufe eine katalysatorreiche Phase und eine produktreiche Phase, die durch an späterer Stelle noch erwähnte geeignete Maßnahmen voneinander getrennt werden können.

Für den Fall, dass die in der mit dem Reaktor verbundenen Trennstufe gebildete produktreiche Phase noch Katalysator enthält, weist das Verfahren einen weiteren Schritt des Austretens der produktreichen Phase aus dieser Trennstufe und des Eintretens der produktreichen und noch Katalysator enthaltenden Phase in eine weitere Trennstufe auf. In dieser weiteren Trennstufe werden vorzugsweise wieder, bevorzugt durch Änderung eines Betriebsparameters oder durch Hinzufügen eines ersten Hilfsstoffes, zumindest zwei Phasen erzeugt, nämlich eine katalysatorreiche und eine produktreiche Phase, wobei die produktreiche Phase bevorzugt keinen Katalysator und alternativ weniger Katalysator als die in der ersten Trennstufe erzeugte produktreiche Phase enthält. In letzterem Fall ist der weiteren Trennstufe noch eine weitere Trennstufe nachgeschaltet, in der wiederum bevorzugt durch die oben genannten Maßnahmen zwei Phasen erzeugt werden, wobei die produktreiche Phase vorzugsweise keinen Katalysator mehr enthält. Bevorzugt passiert die produktreiche und noch Katalysator enthaltende Phase so viele dem Reaktor nachgeschaltete Trennstufen, wie es erfordert, um eine produktreiche Phase zu erzeugen, die im Wesentlichen frei von Katalysator ist.

Das Abtrennen der produktreichen Phase von der katalysatorreichen Phase erfolgt in dem erfindungsgemäßen Verfahren durch bekannte Methoden der Phasentrennung. Beispielsweise kann ein einphasiges, flüssiges Produkt/Katalysator-Gemisch in einer destillationsbasierten Trennung mit einem hochsiedenden Lösungsmittel, z.B. N-Methyl-pyrrolidinon, versetzt werden. In einem Verdampfer werden anschließend die leichtflüchtigen Komponenten, i.d.R. Produkte und etwaige noch enthaltene Reaktanden, als produktreiche Phase in die Gasphase überführt, während das höhersiedende Lösungsmittel mit dem gelösten Katalysator flüssig bzw. flüssiggelöst als katalysatorreiche Phase zurückbleibt. Diese Methode eignet sich besonders für kurzkettige Kohlenwasserstoff-Produkte mit beispielsweise vier Kohlenstoffatomen.

Eine weitere Möglichkeit der Abtrennung der produktreichen Phase von der katalysatorreichen Phase ist eine extraktionsbasierte Trennung, die sich insbesondere für langkettige Kohlenwasserstoff-Produkte eignet. Hier unterscheidet man im Wesentlichen drei Fälle:
Bei der Flüssig-Flüssig-Zweiphasentechnik (FFZP) befinden sich im Reaktor oder in der Trennstufe bereits zwei nicht mischbare Phasen, nämlich eine polare katalysatorreiche Phase, die ein polares Lösungsmittel, z.B. Propylencarbonat, und den Katalysator enthält, und eine unpolare Produktphase, die neben dem Produkt optional auch noch Reaktanden und/oder zusätzliches unpolares Lösungsmittel, z.B. Decan, enthält. Die Trennung der polaren von der unpolaren Phase erfolgt in einer nachgeschalteten Trennstufe mit Beruhigungszone (Abscheider), in der sich die beiden Flüssigkeiten aufgrund ihrer unterschiedlichen Dichte übereinander ansammeln, sodass die untere Flüssigkeit bevorzugt durch eine Öffnung im Boden der Trennstufe abgelassen werden kann.

Das Funktionsprinzip des Temperaturgesteuerten Mehrkomponenten Lösungsmittelsystems (TML) ähnelt stark der Flüssig-Flüssig-Zweiphasentechnik, mit dem Unterschied, dass eine gezielte Ausnutzung bestimmter Phasenzustände in Abhängigkeit der Temperatur stattfindet. Bei Reaktionstemperatur stellt sich im Reaktor oder in der Trennstufe ein einphasiges Gemisch bestehend aus Produkten, Reaktanden, polarem Lösungsmittel (Bsp. DMF) und unpolarem Lösungsmittel (Bsp. Decan) sowie Katalysator ein. Nach einer Temperaturänderung bildet das System zwei flüssige Phasen aus und ermöglicht eine Auftrennung in einem Abscheider.

Bei Verwendung einer nachgeschalteten Extraktion liegt im Reaktor ein einphasiges Gemisch mit den Reaktanden, polarem Lösungsmittel (Bsp. DMF) und Katalysator vor. Nach Ablauf der Reaktion wird die Mischung aus Produkt, Katalysator, polarem Lösungsmittel und ggf. nicht umgesetzten Reaktanden in einen Mischbehälter gegeben, in dem sie mit einem unpolaren Lösungsmittel (Bsp. Decan) versetzt wird. Dadurch wird eine zweite Phase gebildet wird, in der sich bevorzugt Produkte oder Katalysator lösen und die in einem Abscheider von der anderen Phase getrennt wird.

In jeder Ausführungsform des erfindungsgemäßen Verfahrens, in der die Erzeugung und/oder Trennung der katalysatorreichen Phase von der produktreichen Phase in einer dem Reaktor nachgeschalteten Trennstufe erfolgt, weist das Verfahren einen Schritt des Rückführens der katalysatorreichen Phase in den Reaktor auf. Die produktreiche Phase wird dagegen aus der Trennstufe gelassen und ggf. einer mit der Trennstufe verbundenen Aufbereitungsstation zugeführt.

Bei Ausführungsformen, in denen die Erzeugung und/oder Trennung der katalysatorreichen Phase von der produktreichen Phase in mehreren dem Reaktor nachgeschalteten Trennstufen erfolgt, ist es bevorzugt, dass aus jeder dieser Trennstufen die katalysatorreiche Phase austreten gelassen und in den Reaktor zurückgeführt wird, während die produktreiche Phase jeweils von einer Trennstufe in die direkt nachgeschaltete Trennstufe überführt wird und ggf., bevorzugt wenn kein Katalysator mehr enthalten ist, einer Aufbereitungsstation zugeführt wird.

Da der Katalysator durch das erfindungsgemäße Verfahren bevorzugt nicht ausgetragen wird, ist eine Nachdosierung des Katalysators oder seiner Bestandteile während des Verfahrens bevorzugt nicht erforderlich. Es kann jedoch sein, dass für die Bereitstellung und Gewährleistung der Wirkungsweise des Katalysators ein Hilfsstoff, z.B. ein Lösungsmittel, benötigt wird, bei dem es im Laufe der katalysierten Reaktion zu Verlusten kommt. Das erfindungsgemäße Verfahren enthält daher optional eine Schritt, in dem dieser Hilfsstoff, der für die Zwecke der Erfindung als zweiter Hilfsstoff bezeichnet wird, dem Reaktor und/oder einer Trennstufe zugeführt wird, vorzugsweise in einem sogenannten Make-up-Strom. Durch diesen den zweiten Hilfsstoff enthaltenden Make-up-Strom wird eine dauerhafte kontinuierliche Betriebsweise gewährleistet.

Optional liegen der erste Hilfsstoff, der ggf. zur Erzeugung der zumindest zwei Phasen in den Reaktor oder in eine Trennstufe gegeben wird, und der zweite Hilfsstoff als Gemisch vor und werden als Gemisch dem Reaktor oder einer Trennstufe zugegeben. Alternativ dazu sind der erste und der zweite Hilfsstoff optional identisch.

In einer weniger bevorzugten Ausführungsform enthält das Verfahren außerdem einen Schritt des Nachdosierens an Katalysator, z.B. durch Zuführung von neuem Katalysator in den Reaktor. Da der Austrag an Katalysator jedoch in dem erfindungsgemäßen Verfahren jedoch zumindest drastisch minimiert und daher in der Regel verschwindend gering ist oder auch gar nicht auftritt, ist es bevorzugt, auf einen Ersatz eventueller Katalysatorverluste zu verzichten und den ggf. minimalen Austrag an Katalysator im Rahmen der Auswertung zu berücksichtigen.

Generell ist für die Zwecke der Erfindung bei einer Zuführung eines Stoffes in den Reaktor oder eine Trennstufe auch eine Zuführung in eine mit dem Reaktor oder der Trennstufe verbundene Leitung gemeint, die dann in dem jeweiligen Behälter mündet.

Das erfindungsgemäße Verfahren weist weiterhin einen Schritt der Untersuchung des Katalysators, z.B. auf Alterungsphänomene oder Vergiftungsphänomene, auf. Bei dieser Untersuchung des Katalysators werden die Eigenschaften des Katalysators zu einem Zeitpunkt x nach Beginn der durch den Katalysator katalysierten Reaktion mit den entsprechenden Eigenschaften des Katalysators vor Beginn der Reaktion, d.h. z.B. bei Zuführung des Katalysators in den Reaktor, verglichen.

Bei den zu vergleichenden Eigenschaften handelt es sich insbesondere um die Zusammensetzung und Struktur des Katalysators, um seine räumliche Ausrichtung und/oder um physikalische Eigenschaften wie Molekulargewicht, Dichte, Schmelz- oder Siedepunkt. Eine weitere Eigenschaft des Katalysators, die in dem erfindungsgemäßen Verfahren untersucht werden kann, ist dessen Katalyseleistung. Dazu wird die Katalyseleistung des frisch in den Reaktor eingebrachten Katalysators mit dessen Katalyseleistung zu einem Zeitpunkt nach Beginn der Reaktion verglichen, z.B. in dem die Menge der in einem bestimmten Zeitraum umgesetzten Edukte oder der in diesem Zeitraum gebildeten Produkte verglichen wird. Erfindungsgemäß wird zumindest eine Eigenschaft des Katalysators vor Beginn der Reaktion mit der entsprechenden Eigenschaft nach Beginn der Reaktion verglichen. Da die Reaktion in dem Reaktor bevorzugt für mehrere Tage bis Wochen kontinuierlich abläuft, liegt der Zeitpunkt x, zu dem die zumindest eine Eigenschaft des Katalysators untersucht wird, bevorzugt mindestens drei Tage nach der erstmaligen Zuführung des Katalysators in den Reaktor. Optional weist das Verfahren im Laufe der Reaktion auch mehrere zeitlich voneinander beabstandete Schritte der Untersuchung des Katalysators in zumindest einer Eigenschaft auf, z.B. nicht nur vor der Reaktion und einmal im Laufe der Reaktion, sondern z.B. vor der Reaktion sowie nach 3, 6, 9, 12, und 15 Tagen oder z.B. einmal oder mehrmals wöchentlich für mehrere Wochen, oder nach jeweils einer bestimmten Anzahl von Katalysezyklen, beispielsweise nach jeweils 100, 500 oder 1000 Katalysezyklen.

Aus dem Vergleich der zumindest einen Eigenschaft des Katalysators zum Zeitpunkt vor der Reaktion und zum Zeitpunkt x im Laufe der Reaktion können bevorzugt Rückschlüsse auf die Stabilität, insbesondere die Langzeitstabilität des Katalysators, auf dessen Langzeitleistungsfähigkeit sowie auf dessen Eignung zur Katalyse einer bestimmten Reaktion und/oder auf dessen Anfälligkeit für Alterungs- und/oder Vergiftungserscheinungen gezogen werden.

In einer bevorzugten Ausführungsform des Verfahrens ist die katalysierte Reaktion eine Hydroformylierungsreaktion. Entsprechen sind die eingesetzten Reaktanden Kohlenstoffmonoxid, Wasserstoff und zumindest eine Olefinverbindung.

Die erfindungsgemäße Anlage ist für die Durchführung eines erfindungsgemäßen Verfahrens eingerichtet. Entsprechend weist sie einen Reaktor mit zumindest einer Einlassöffnung und zumindest einer Auslassöffnung auf. Alternativ weist die Anlage auch mehrere solcher Reaktoren auf, wobei jeder Reaktor mit zumindest einer Einlassöffnung über eine Leitung mit zumindest einer Auslassöffnung eines vorgeschalteten Reaktors verbunden ist, sofern es einen vorgeschalteten Reaktor gibt. Der zumindest eine Reaktor der Anlage ist mit seiner Auslassöffnung, ggf. über mehrere zwischengeschaltete weitere Reaktoren, mit der Einlassöffnung einer Trennstufe verbunden. Optional sind dieser Trennstufe weitere Trennstufen nachgeschaltet.

Die zumindest eine Trennstufe ist über eine zweite Leitung mit dem Reaktor verbunden, durch welche der Katalysator in den Reaktor zurückgeführt wird. Bevorzugt weisen der zumindest eine Reaktor und die zumindest eine Trennstufe jeweils Zuleitungen auf, über die Lösungsmittel, erster Hilfsstoff und/oder zweiter Hilfsstoff in den Reaktor oder in die Trennstufe eingebracht werden können.

Da die Anlage zur Durchführung des erfindungsgemäßen Verfahrens eingerichtet ist, soll die Beschreibung des erfindungsgemäßen Verfahrens auch als Beschreibung der Anlage zur Durchführung des Verfahrens gelten.

Die Erfindung wird nun genauer anhand von Beispielen und mit Bezug auf die Figuren beschrieben, in denen schematisch
- Figur 1 eine Übersicht möglicher Stromführungen in dem erfindungsgemäßen Verfahren zeigt,
- Figur 2 eine Darstellung einer erfindungsgemäßen Anlage zeigt,
- Figur 3 eine bevorzugte Stromführung in dem erfindungsgemäßen Verfahren zeigt,
- Figur 4 eine weitere bevorzugte Stromführung in dem erfindungsgemäßen Verfahren zeigt, und
- Figur 5 eine weitere bevorzugte Stromführung in dem erfindungsgemäßen Verfahren zeigt.

In den Figuren bezeichnen gleiche Bezugsziffern funktionsgleiche Teile.

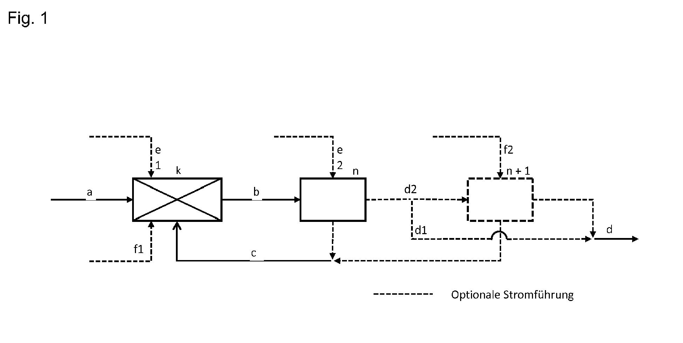

Die Figur 1 zeigt eine Übersicht über die möglichen Stromführungen in einem erfindungsgemäßen Verfahren mit zumindest einer Trennstufe n, die dem Reaktor k nachgeschaltet ist. In der Übersicht sind notwendige Stromführungen mit einer durchgängigen Linie und optionale Stromführungen mit einer gestrichelten Linie gekennzeichnet.

Die Reaktanden 1 werden mit dem Strom a dem Reaktor k zugeführt. Optional enthält dieser Strom außerdem den Katalysator 2, bevorzugt wird dieser jedoch separat hinzugegeben. Nach erfolgter Reaktion oder während der Reaktion in dem Reaktor k werden Produkte und Katalysator 2 mit dem Strom b von dem Reaktor k in die Trennstufe n überführt, optional nachdem durch Zuführung eines ersten Hilfsstoff enthaltenden Stromes e1 zwei Phasen in dem Reaktor erzeugt wurden. Spätestens in der Trennstufe n werden zumindest zwei Phasen erzeugt, nämlich eine katalysatorreiche Phase 3 und eine produktreiche Phase 4. Optional erfolgt die Ausbildung der zwei Phasen dabei aufgrund der Zuführung eines ersten Hilfsstoffes 5, beispielsweise mittels eines ersten Hilfsstoff 5 enthaltenden Stromes e2, der der Trennstufe n zugeführt wird. Nach der Ausbildung bzw. Erzeugung der zumindest zwei Phasen wird die katalysatorreiche Phase 3 mit dem Strom c in den Reaktor k zurückgeführt. Die produktreiche Phase 4 wird entweder mittels des Stromes d1 aus der Trennstufe n austreten gelassen und optional einer Aufbereitungsstation zugeführt oder mittels des Stromes d2 in eine weitere Trennstufe n+1 eintreten gelassen, aus der nach der Erzeugung zweier Phasen die produktreiche Phase 4 mittels des Stromes d austreten gelassen wird.

Die in der weiteren Trennstufe n+1 gebildete katalysatorreiche Phase 3 wird ebenfalls in den Reaktor k zurückgeführt. Dort steht der Katalysator 2 für die weitere Katalyse der mittels des Stromes a neu in Reaktor k eingebrachten Reaktanden 1 zur Verfügung. Optional wird noch ein einen zweiten Hilfsstoff 6 enthaltender Make-up-Strom f1 in den Reaktor k oder eine Trennstufe n eingespeist.

Beispiel 1: Untersuchung der Langzeiteigenschaften homogener Katalysatorsysteme für die

### Hydroformylierung im kontinuierlichen Betrieb

Die Hydroformylierung zählt zu den meist genutzten homogen-katalysierten Reaktionen der chemischen Industrie, bei denen Olefine mit Hilfe von Synthesegas (CO/H₂) zu Aldehyden umgesetzt werden können. Zur Beurteilung der Leistungsfähigkeit neuartiger Katalysatorsysteme, z.B. basierend auf verschiedenen Übergangsmetallen (Rh, Ir, Ru, etc.) und/oder bekannten oder innovativen Liganden, ist oftmals notwendig, die Langzeitstabilität bzw. -verhalten dieser Systeme im kontinuierlichen Maßstab zu untersuchen.

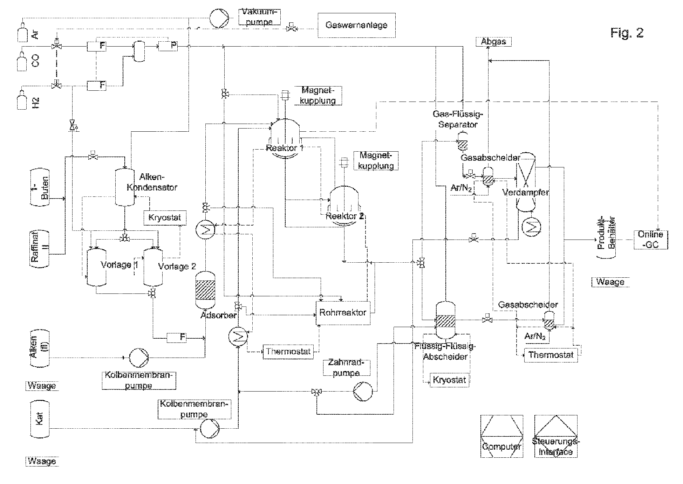

Um die Kosten der Untersuchung zu minimieren, wird eine Prozessanordnung im Miniplant-Maßstab (Durchsatz: 0,01 - 1 l/h) eingesetzt. Figur 2 stellt das Verfahrensfließbild der verwendeten Prozessapparatur detailliert dar, die die in Figur 1 gezeigte konzeptionelle Superstruktur extraktions- oder/und destillationsbasiert realisiert.

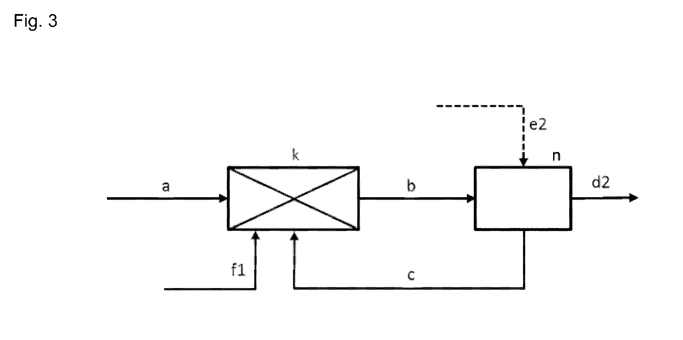

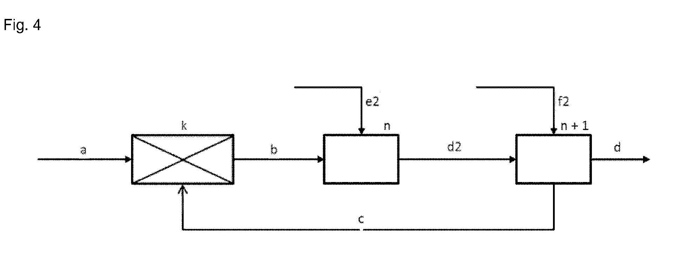

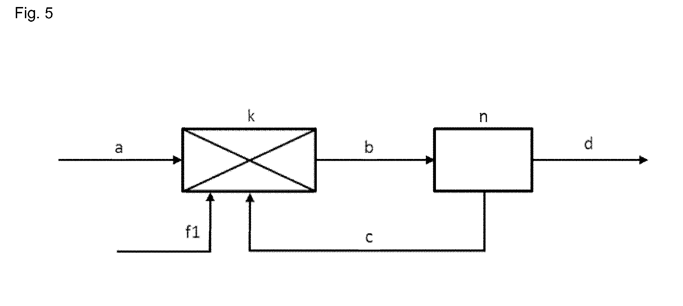

Die überschaubare und doch flexible Anlage ist multifunktional und ermöglicht die Untersuchung dreier unterschiedlicher Operationskonzepte, und zwar gemäß I. mit Einspeisung des Make-up-Stromes des zweiten Hilfsstoffes 6 in den Reaktor k (Figur 3), und gemäß II. unter Verwendung einer zusätzlichen Rückgewinnungstrennstufe n+1, die den Make-up-Strom des zweiten Hilfsstoffes 6 nutzt (Figur 4) und (III.) unter Verwendung einer thermischen Trennung mit Verzicht auf einen zusätzlichen ersten Hilfsstoff 5 (Figur 5).

Gemäß Figur 3 werden die Reaktanden 1 mit dem Strom a in den Reaktor k eintreten gelassen. Dem Reaktor k wird außerdem Katalysator 2 zugeführt. Während und/oder nach der Reaktion der Reaktanden 1 in dem Katalysator 2 wird das einphasige oder mehrphasige Gemisch, das den Katalysator 2 und Produkt enthält, mittels des Stromes b aus dem Reaktor k in die Trennstufe n überführt. Mittels des Stromes e2 wird der Trennstufe außerdem ein erster Hilfsstoff 5 zugeführt, welcher die Bildung einer neuen Phase initiiert oder unterstützt. In der Trennstufe n werden daher eine katalysatorreiche Phase 3 und eine produktreiche Phase 4 erzeugt. Die produktreiche Phase 4 wird mit dem Strom d aus der Trennstufe n austreten gelassen, die katalysatorreiche Phase 3 wird mit dem Strom c in den Reaktor k zurückgeführt. Mittels des Stromes f1 wird ein einen zweiten Hilfsstoff 6 enthaltender Make-up-Strom in den Reaktor k eingebracht, wobei es sich bei dem zweiten Hilfsstoff 6 z.B. um Lösungsmittel für den Katalysator 2 handelt.

Eine alternative Stromführung in einer weiteren Ausführungsform des erfindungsgemäßen Verfahrens ist in der Figur 4 gezeigt. Analog zu der in Figur 3 gezeigten Stromführung werden dem Reaktor k Reaktanden 1 und Katalysator 2 zugeführt und die Produkt, Katalysator 2 und optional nicht umgesetzte Reaktanden 1 enthaltende Mischung wird mittels des Stromes b der Trennstufe n zugeführt. Unter Zuführung eines einen ersten Hilfsstoff 5 enthaltenden Stromes e2 werden in der Trennstufe n zwei Phasen ausgebildet. Die katalysatorreiche Phase 3 und die produktreiche Phase 4 werden jedoch nicht direkt in dieser Trennstufe n, sondern erst in einer nachgeschalteten Trennstufe n+1 voneinander getrennt, wobei die produktreiche Phase 4 aus der Trennstufe n+1 austreten gelassen wird und die katalysatorreiche Phase 3 von der Trennstufe n+1 in den Reaktor k zurückgeführt wird. Vor, während oder nach des Austretens der produktreichen Phase 4 und der katalysatorreichen Phase 3 aus der Trennstufe n+1 wird dieser mittels des Stromes f2 ein einen zweiten Hilfsstoff 6 enthaltender Make-up-Strom zugeführt. Dieser kann optional auch die Ausbildung der unterschiedlichen Phasen initiieren oder unterstützen. Die mittels des Stromes d aus der Trennstufe n+1 austretende produktreiche Phase 4 ist vorzugsweise frei von Katalysator 2.

In der in Figur 5 gezeigten Ausführungsform des erfindungsgemäßen Verfahrens gelangen die Reaktanden 1 mit dem Strom a in den Reaktor k, dem auch der Katalysator 2 zugeführt wird. Nach oder während der durch den Katalysator 2 katalysierten Reaktion der Reaktanden 1 tritt das optional ein- oder mehrphasige Gemisch, das den Katalysator 2 und das Produkt enthält, in die in dieser Ausführungsform einzige Trennstufe n über. Für den Fall, dass das Gemisch mehrphasig ist und eine katalysatorreiche Phase 3 und eine produktreiche Phase 4 enthält, werden diese voneinander getrennt, wobei die produktreiche Phase 4 mittels des Stromes d aus der Trennstufe n austreten gelassen wird und die katalysatorreiche Phase 3 mittels des Stromes c wieder in den Reaktor k zurückgeführt wird. Für den Fall, dass das mittels des Stromes b in die Trennstufe n überführte Gemisch mit dem Katalysator 2 und dem Produkt einphasig ist, sieht diese Ausführungsform die Änderung zumindest eines Betriebsparameters vor, die zur Bildung einer zweiten Phase führt, in welcher sich Katalysator 2 oder Produkt anreichert. Diese Änderung zumindest eines Betriebsparameters kann beispielsweise eine Änderung der Temperatur oder eine Änderung des Druckes in der Trennstufe n einschließen. Nach Erzeugung der zumindest zwei Phasen werden die katalysatorreiche Phase 3 und die produktreiche Phase 4 voneinander getrennt, wobei erstere mittels des Stromes c in den Reaktor k zurückgeführt wird und letztere mittels des Stromes d aus der Trennstufe n austreten gelassen wird.

In jeder der in den Figuren gezeigten Ausführungsformen weist das erfindungsgemäße Verfahren den Schritt des Untersuchens des Katalysators 2 auf, der einen Vergleich zumindest einer Eigenschaft des Katalysators 2 vor der Reaktion mit dessen entsprechender Eigenschaft nach einer Vielzahl von Katalysezyklen beinhaltet, wobei der Grad der Abweichung der untersuchten Eigenschaften voneinander es erlaubt, Aussagen über die Langzeiteigenschaften des Katalysators 2 zu treffen. Diese Aussagen über die Langzeiteigenschaften des Katalysators 2 sind umso aussagekräftiger, je größer die Zeitspanne zwischen dem Start der katalytischen Reaktion in dem Reaktor k, d.h. der ersten Untersuchung auf zumindest eine Eigenschaft, und dem Zeitpunkt der zweiten Untersuchung auf die entsprechende Eigenschaft ist.

Wesentliche Aspekte der Erfindung sollen nochmal zusammengefasst werden: Das Verfahren zur Untersuchung der Langzeiteigenschaften homogener Katalysatorsysteme im kontinuierlichen Betrieb weist die folgenden Schritte auf: Einspeisen von Reaktanden in einen Reaktor; Zuführen einer definierten Ausgangsmenge Katalysator in den Reaktor; Erzeugen von mindestens zwei Phasen, umfassend eine katalysatorreiche Phase und eine produktreiche Phase; Abtrennen der produktreichen Phase von der katalysatorreichen Phase; Untersuchung des Katalysators, wobei die Untersuchung einen Vergleich zumindest einer Eigenschaft des Katalysators zum Zeitpunkt der erstmaligen Zuführung in den Reaktor und zu einem späteren Zeitpunkt beinhaltet.

### Bezugszeichenliste

- 1: Reaktanden
- 2: Katalysator
- 3: katalysatorreiche Phase
- 4: produktreiche Phase
- 5: erster Hilfsstoff
- 6: zweiter Hilfsstoff
- 7: Einlassöffnung des Reaktors
- 8: Auslassöffnung des Reaktors
- 9: Einlassöffnung der Trennstufe
- 10: Auslassöffnung der Trennstufe
- 11: erste Leitung zwischen dem Reaktor und der Trennstufe
- 12: zweite Leitung zwischen dem Reaktor und der Trennstufe
- k: Reaktor
- n: Trennstufe
- n+1: weitere Trennstufe
- a: Reaktanden enthaltender Strom
- b: Produkt und Katalysator enthaltender Strom
- c: Katalysator enthaltender Strom
- d: Produkt enthaltender Strom
- e: ersten Hilfsstoff enthaltender Strom
- f: zweiten Hilfsstoff enthaltender Strom

## Patentansprüche

1. Verfahren zur Untersuchung der Langzeiteigenschaften homogener Katalysatorsysteme im kontinuierlichen Betrieb mit den Schritten
a) Einspeisen von Reaktanden (1) in einen Reaktor (k),
b) Zuführen einer definierten Ausgangsmenge Katalysator (2) in den Reaktor (k),
c) Erzeugen von mindestens zwei Phasen, umfassend eine katalysatorreiche Phase (3) und eine produktreiche Phase (4),
d) Abtrennen der produktreichen Phase (4) von der katalysatorreichen Phase (3), **dadurch gekennzeichnet, dass** das Verfahren außerdem einen Schritt e) aufweist, der eine Untersuchung des Katalysators (2) beinhaltet, wobei die Untersuchung des Katalysators (2) einen Vergleich zumindest einer Eigenschaft des Katalysators (2) zum Zeitpunkt der Zuführung in den Reaktor (k) unter Schritt b) und zu einem späteren Zeitpunkt beinhaltet.

2. Verfahren nach Anspruch 1, **dadurch gekennzeichnet, dass** die Abtrennung der produktreichen Phase (4) von der katalysatorreichen Phase (3) in dem Reaktor (k) erfolgt und die katalysatorreiche Phase (3) in diesem verbleibt, während die produktreiche Phase (4) aus dem Reaktor (k) austreten gelassen wird.

3. Verfahren nach Anspruch 1, **dadurch gekennzeichnet, dass** die Abtrennung der produktreichen Phase (4) von der katalysatorreichen Phase (3) in einer von optional mehreren dem Reaktor (k) nachgeschalteten Trennstufen (n, n+1) erfolgt, wobei die katalysatorreiche Phase (3) nach der Abtrennung aus der Trennstufe (n, n+1) in den Reaktor (k) zurückgeführt wird.

4. Verfahren nach einem der voranstehenden Ansprüche, **dadurch gekennzeichnet, dass** die produktreiche Phase (4) im Wesentlichen frei von Katalysator (2) ist.

5. Verfahren nach einem der voranstehenden Ansprüche, **dadurch gekennzeichnet, dass** das Erzeugen der katalysatorreichen Phase (3) und der produktreichen Phase (4) durch Hinzufügen eines ersten Hilfsstoffes (5) in den Reaktor (k) und/oder in eine dem Reaktor (k) nachgeschaltete Trennstufe (n, n+1) erfolgt.

6. Verfahren nach einem der voranstehenden Ansprüche, **dadurch gekennzeichnet, dass** das Erzeugen der katalysatorreichen Phase (3) und der produktreichen Phase (4) durch Änderung zumindest eines Betriebsparameters erfolgt.

7. Verfahren nach einem der voranstehenden Ansprüche, **gekennzeichnet durch** Einspeisung eines Make-up Stromes enthaltend einen zweiten Hilfsstoff (6) in den Reaktor (k) und/oder in eine dem Reaktor (k) nachgeschaltete Trennstufe (n, n+1).

8. Verfahren nach einem der voranstehenden Ansprüche, **dadurch gekennzeichnet, dass** der erste Hilfsstoff (5) und der zweite Hilfsstoff (6) als Gemisch dem Reaktor (k) und/oder einer dem Reaktor (k) nachgeschalteten Trennstufe (n, n+1) zugeführt werden.

9. Verfahren nach einem der voranstehenden Ansprüche, **dadurch gekennzeichnet, dass** der spätere Zeitpunkt, zu dem die zumindest eine Eigenschaft des Katalysators (2) mit dessen entsprechender Eigenschaft während der Zuführung in den Reaktor (k) unter Schritt b) verglichen wird, mindestens drei Tage nach der Zuführung in den Reaktor (k) unter Schritt b) liegt.

10. Verfahren nach einem der voranstehenden Ansprüche, **dadurch gekennzeichnet, dass** der Schritt des Abtrennens der produktreichen Phase (4) von der katalysatorreichen Phase (3) eine Destillation und/oder Extraktion einschließt.

11. Verfahren nach einem der voranstehenden Ansprüche, **dadurch gekennzeichnet, dass** der Schritt des Abtrennens der produktreichen Phase (4) von der katalysatorreichen Phase (3) in mehreren Stufen erfolgt.

12. Verfahren nach einem der voranstehenden Ansprüche, **dadurch gekennzeichnet, dass** die Reaktanden (1) Kohlenstoffmonoxid, Wasserstoff und zumindest eine Olefinverbindung sind und die katalysierte Reaktion eine Hydroformylierungsreaktion ist.

13. Verfahren nach einem der voranstehenden Ansprüche, **dadurch gekennzeichnet, dass** eine von einer produktreichen Phase (4) abgetrennte katalysatorreiche Phase (3) aus jeder von mindestens zwei Trennstufen (n, n+1) entfernt und in den Reaktor (k) zurückgeführt wird.

14. Anlage für die Durchführung eines Verfahrens nach einem der voranstehenden Ansprüche mit den folgenden Einrichtungen:
a) mindestens einem Reaktor (k) mit mindestens einer Einlassöffnung (7) und einer Auslassöffnung (8)
b) mindestens einer Trennstufe (n) mit mindestens einer Einlassöffnung (9) und einer Auslassöffnung (10), wobei die Trennstufe (n) über zwei Leitungen (11, 12) mit dem Reaktor (k) verbunden ist.

15. Verwendung der Anlage nach Anspruch 14 zur Durchführung eines Verfahrens nach einem der Ansprüche 1 bis 13.
